# EUROPEAN PATENT APPLICATION

(11) **EP 2 700 944 A2**
(43) Date of publication of application: **26.02.2014**
(21) Application number: 12190511.1
(22) Date of filing: 30.10.2012
(51) Int. Cl.: G01N 33/50

(54) **A method for determining endogenous and exogenous markers in blood**

(30) Priority: 21.08.2012 KZ 20120919
(71) Applicant: Gareyev, Rauf, 050036 Almaty (KZ)
(72) Inventor: Gareyev, Rauf, 050036 Almaty (KZ)
(74) Representative: Lapienis, Juozas

(57) **Abstract**

The method of the invention comprises preparing for analysis of a solution which contains substances, blood plasma, plus those substances which were present in whole blood within an adsorbed layer (were adsorbed) on blood cell surface. The method of the invention characterized in that substances which facilitate the transfer of the substances adsorbed on blood cell surface into the plasma are added to the whole (initial) blood sample. In accordance with the most preferred embodiment, a sodium chloride hypertonic solution or a certain amount of a dry salt powder of sodium chloride is added.

The method of the invention is intended for the analysis of all of the currently detectable endogenous substances and exogenous substances in blood but, in the first turn, for conducting pharmacological studies as well as for detecting tumor markers, immunomarkers, and doping control markers, which are transported in blood mostly on the blood cell surface.

## Description

The invention relates to the field of medicine and biomedical research, and more particularly to the analysis of endogenous substances and exogenous substances carried by the blood, and even more particularly to the determination of tumor markers and markers for doping control in the blood, and the use of blood samples in other laboratory analyses as well as in pharmacokinetic and other studies.

### Prior Art

Currently, laboratory methods of human blood tests are widely used for doping control markers detection in sportsmen, for definition of pharmacological preparations effect on experimental animals and humans, for cancer and other diseases diagnostics. In these tests biomarkers level in blood is determined from their level in plasma.

### Prototype, Weak Points

These tests, however, have certain disadvantages. For example, some drugs are not detected in blood plasma. They are, therefore, only estimated in relation to their metabolites in urine or hair follicles (Reiter et al., Doping Journal, v.5, 3, 2008, and others). Often tumor markers confirm a diagnosis at an advanced stage of the disease. Some chronic infectious and parasitic diseases are not confirmed by antigen-antibody complex formation tests. Both mechanism and underlying cause of allergic and autoimmune processes are not always clear from immunoglobulin determination data. This may be due to the fact that IgD, IgE, IgM are carried on the surface of white blood cells.

Some of the above listed disadvantages are associated directly with a complex of erythrocyte properties detected by us and referred to as adsorption-transport function of erythrocytes.

Our studies have shown that many biomarkers are carried mostly on the blood cell surface. These substances adsorbed on the blood cell surface are transported directly into the exchange layer of blood capillaries where they participate, in the first place, in both transcapillary metabolism and tissue metabolism. For many biomarkers, the indicators of their transport on the erythrocytes surface are more informative than their plasma level data used in practice (see 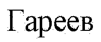 P.A. 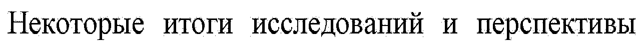 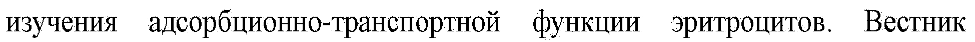 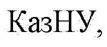

Biology Series, Nº2, 2010, p. 103-109; Gareyev R. Adsorption-Transport Function of Erythrocytes: Important Facts about New Diagnostic Capability. International Journal of Applied and Fundamental Research. No. 1, 2011, p. 5-10).

According to our data, many biomarkers are "thrown off' from the blood cells surface into plasma by stress. Therefore some biomarkers level in plasma varies within wide limits. The total content index of each certain substance in plasma and among substances adsorbed on the blood cell surface generally has a substantially (1.5 to 2 times) lower variation ratio (a data scattering index) than the corresponding index for plasma only.

There are known methods for isolating substances which are adsorbed on the surface of erythrocytes into a solution (see RK Provisional Patents Nos. 16018, 16019, 17280 to R. A. Gareyev, and others). However, the methods provided are not unified and comprise a complicated two-stage or multiple-stage process of preparing samples for analyses.

The number of erythrocytes is about 2 to 3 orders of magnitude greater than the other blood cells. Therefore, we designate the substances which are adsorbed on blood cell surface as erythrocyte-adsorbed substances (substances adsorbed on the surface of erythrocytes or substances of an erythrocyte-adsorbed pool). Such designation is based on the methods disclosed in the above provisional patents which make it possible to analyze substances adsorbed exclusively on the surface of erythrocytes. In this manner, the substances adsorbed on the surface of leukocytes, and thrombocytes do not enter the solution being prepared for the analysis. Furthermore, the methods disclosed in the above provisional patents do not take account for change in an erythrocyte volume in the process of removing the erythrocyte-adsorbed substances into an eluate (a washing solution) or into plasma.

### Essence of the Invention

The present invention suggests to use a substrate containing all of the extracellular blood markers, i.e., plasma markers plus markers carried on the blood cell surface for all the required research, wherein blood plasma (serum) is currently analyzed. For this purpose, the present invention provides a method of a single-stage removal of biomarkers on thrombocytes, erythrocytes, and leukocytes into plasma (or a mixture of plasma and added solution and cells filtrate). The mixture of substances obtained is a biomaterial (sample) for the detection of the required endogenous and exogenous blood markers.

### Practicability Data

The method in accordance with the present invention differs from conventional blood cell washing methods (Kikuchi, Y. & Koyama T. Red blood cell deformability and protein absorption on red blood cell surface. Am J Physiol. 1984, v. 247, pp. 739-747, 1672) in a number of aspects. The method in accordance with the present invention is based on an additional effect of washing. Such effect is associated with the filtration of a portion of intracellular water paralleled by a reduction in erythrocyte and leukocyte volume. The filtration is caused by an increase in intracellular osmotic pressure following the addition of a salt or a hypertonic salt solution (HSS) to blood in accordance with the present invention.

The osmotic pressure of a solution being added depends on both concentration and properties of salts contained therein. Sodium chloride has nearly no contraindication for subsequent analyses. The concentration of a NaCl hypertonic salt solution added may be of between about 2-6%. The resistance of erythrocytes against hypertonic solutions is variable. It is especially variable in sick and elderly people. As the reference solution, we, therefore, recommend using a 4% NaCl solution. In such cases, hemolysis has been reported relatively rare. The volume of a hypertonic solution added to blood depends on the sensitivity of the analysis method intended. It is common knowledge that the higher blood dilution with a solution added, the lower the concentration of biomarkers in the substrate analyzed.

An alternative of adding a dry powder of sodium chloride to a blood sample requires cautious stirring. An alternative in which a blood sample is passed through a tube having a metered salt content on the interior wall is more acceptable. Alternatives in which test strips are employed are possible as well.

In individual special studies, washing is possible using a solution of large molecule compounds which increase blood oncotic pressure. In other special studies, substances, for example, stress hormones such as adrenaline etc. that also facilitate the transfer of substances adsorbed on blood cells into plasma may be employed.

In accordance with yet another alternative embodiment, the present invention provides taking the whole plasma following blood centrifugation. Instead of plasma, a hypertonic solution is added (in the volume of plasma taken) to the erythrocyte mass. The erythrocytes and the solution added are then mixed, and the mix so obtained is again centrifuged. This supernatant solution and plasma are used as samples for the relevant analyses.

In all the cases, account is taken of the dilution of the initial volume of plasma with the solution added and water which leaves blood cells following the addition of a hypertonic solution or salt. For more accurate corrections, hematocrit of the initial blood and that following the addition of a substance which facilitates the transfer of substances adsorbed on red blood cells into plasma are determined. From these hematocrits, the correction for the volume of erythrocyte mass changed is calculated.

Example: One ml of blood was mixed with 1 ml of a 4% sodium chloride (NaCl) solution. The mixture so obtained was centrifuged. In the supernatant liquid, protein was detected in a concentration of 31 g/l using the standard biuret test. The hematocrit (Ht) of venous blood was 45%; accordingly, the volume of plasma was 55% or 0.55 ml per 1 ml of blood. In the 4% NaCl hypertonic solution, the volume of erythrocyte mass reduced by a third (by 0.15 ml). Thus, as compared with the initial blood sample, the volume of "plasma" (0.55 ml of plasma plus 1 ml of the 4% NaCl solution plus 0.15 ml of water which left the erythrocytes) increased 3.09 times as much (0.55+1.0+0.15/0.55=3.09). By multiplying the supernatant concentration of protein (31 g/l) by this dilution factor, we obtain (31*3.09=95.79). The protein concentration in plasma was 65 g/l; the difference (95.79-65=30.75) is protein located on blood cell surface.

The substances adsorbed on blood cell surface along with plasma substances are extracellular substances (markers).

The calculations are not a subject of patenting. It should be noted, however, that it is more correctly to calculate the total level of any extracellular blood marker per volume unit of blood rather than per volume unit of plasma. In such a case, a figure obtained depends on a lower extent of food intake, stress, dewatering, and many other exposures and conditions. Upon addition of three (and more) volumes of hypertonic solution to one blood volume, the erythrocyte volume fraction of the mixture is negligible. Thereby, the calculation is simplified.

For example, the supernatant solution contains 14.3 g/l of protein. By multiplying 14.3 g/l by the total volume of blood and solution added (1 + 3=4) (14.3*4=57.2), we obtain 57.2 g/l per volume unit (1) of blood. If this figure is "contracted" to the initial volume of plasma (0.55), we obtain (57.2/0.55=104 g/l) 104 g/l. This figure (104) is more than 95.79 only by 8.6%. For production studies, their respective correction factor must be derived.

The method in accordance with the present invention is intended for the analysis of all of the currently detectable endogenous substances and exogenous substances in blood but, in the first turn, of tumor markers, immunomarkers, markers for doping control, and other biomarkers carried in the blood mostly on blood cell surface. The method of the invention is compatible with all of the laboratory techniques including PCR-based diagnostics, immune, immune-enzyme, and immunoradioactive assays.

## Claims

1. A method for determining various endogenous substances and exogenous substances in the blood, the method comprising: taking blood; centrifuging the blood; taking a supernatant solution; determining concentrations of the substances tested contained in the supernatant solution using known methods, **characterized in that** the solution prepared for analysis contains substances which were present in blood plasma and substances which were present in whole blood within an adsorbed layer (were adsorbed) on blood cell surface.

2. The method as claimed in claim 1, **characterized in that** substances which facilitate the transfer of the substances adsorbed on blood cell surface into the plasma are added to the whole (initial) blood sample.

3. The method as claimed in claim 1, **characterized in that** the added substance comprises generally sodium chloride or a washing hypertonic solution (eluate) and other substances which increase the transfer of the substances adsorbed on blood cell surface into the plasma.
